(19) Europäisches Patentamt
European Patent Office
Office européen des brevets

(11) Veröffentlichungsnummer: **0 014 411**
**B1**

(12) # EUROPÄISCHE PATENTSCHRIFT

(45) Veröffentlichungstag der Patentschrift: **07.10.81**

(51) Int. Cl.³: **C 07 D 405/14**, C 07 D 405/04 // C07D403/12, C07D235/12

(21) Anmeldenummer: **80100429.2**

(22) Anmeldetag: **28.01.80**

(54) **Verfahren zur Herstellung von Benzimidazolylbenzofuranen.**

(30) Priorität: **08.02.79 DE 2904829**

(43) Veröffentlichungstag der Anmeldung: **20.08.80 Patentblatt 80/17**

(45) Bekanntmachung des Hinweises auf die Patenterteilung: **07.10.81 Patentblatt 81/40**

(84) Benannte Vertragsstaaten: **CH DE FR GB IT**

(56) Entgegenhaltungen:

**EP A-0 010 063**

**JOURNAL OF THE CHEMICAL SOCIETY, April 1960, Seiten 1954–1955 London, G.B. K.B.L. MATHUR et al.: «Preparation of 2-p-Nitrophenylbenzofuran»**

(73) Patentinhaber: **BAYER AG, Zentralbereich Patente, Marken und Lizenzen, D-5090 Leverkusen 1, Bayerwerk (DE)**

(72) Erfinder: **Wehling, Bernhard, Dr., Andreas-Gryphius-Strasse 26, D-5000 Koeln 80 (DE)**
Erfinder: **Bremen, Josef, Dr., Am Kettnersbusch 1 e, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 014 411 B1

ACTORUM AG.

# Verfahren zur Herstellung von Benzimidazolylbenzofuranen

Gegenstand der Erfindung ist ein Verfahren zur Herstellung von Verbindungen der Formel

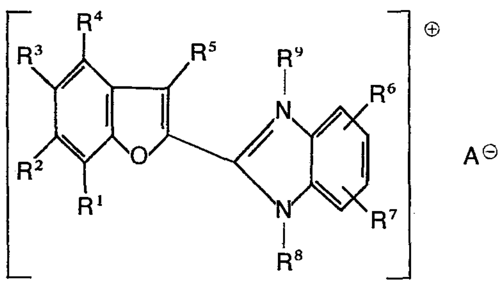

(I)

welches dadurch gekennzeichnet ist, dass man eine Verbindung der Formel

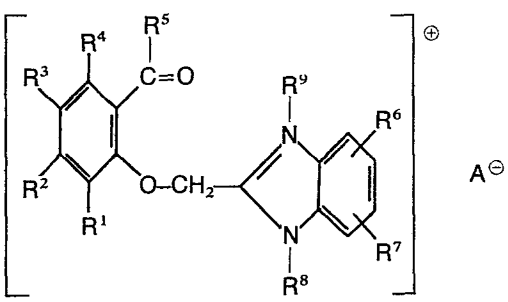

(II)

in einem inerten organischen Lösungsmittel ohne Zugabe eines Katalysators erhitzt.

In den allgemeinen Formeln (I) und (II) bedeuten:

$R^1$, $R^3$, $R^4$ Wasserstoff, Alkyl, Alkoxy oder Halogen,

$R^2$ Wasserstoff, Halogen, Alkyl, Alkoxy oder Aryl oder einen Substituenten der allgemeinen Formel

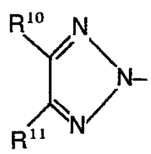

in der

$R^{10}$ Aryl, Alkyl, Aralkyl, Styryl oder Alkoxy,

$R^{11}$ Wasserstoff, CN, COOR, CONRR' oder $R^{10}$ und zwei benachbarte

$R^1$, $R^2$, $R^3$, $R^4$, $R^{10}$, $R^{11}$ zusammen einen anellierten hydroaromatischen oder aromatischen Ring,

$R^5$ Wasserstoff, Aryl, Alkyl,

$R^6$ Wasserstoff, Halogen, Alkyl, Alkoxy, Alkylsulfonyl, Arylsulfonyl, CN, $CF_3$, COOR, $SO_3R$, CONRR' oder $SO_2NRR'$,

$R^7$ Wasserstoff, Halogen, Alkyl, Alkoxy,

$R^8$ Wasserstoff, Alkyl, Cycloalkyl, Alkenyl, Aryl oder Aralkyl,

$R^9$ Cycloalkyl, Alkenyl, Alkyl, Aralkyl,

$A^{\ominus}$ ein farbloses Anion einer anorganischen oder organischen Säure,

R Alkyl und

R' R oder H, wobei die vorstehend genannten Kohlenwasserstoff- und Alkoxyreste sowie die gegebenenfalls anellierten Ringsysteme durch in der Aufhellerchemie übliche nichtionische Substituenten substituiert sein können.

Geeignete beliebige Alkyl- und Alkoxyreste haben 1–4 C-Atome.

Geeignete Arylreste sind Phenylreste. Geeignete nichtionische Substituenten sind Alkyl, Alkoxy, Halogen, CN, COOR, $SO_2R$ u.a.m. Unter «Halogen» wird vor allem Brom und insbesondere Chlor verstanden.

Geeignete Alkenylreste weisen 3–5 C-Atome auf.

Geeignete Cycloalkylreste sind Cyclohexylreste.

Geeignete Ringe, die $R^{10}$ unc $R^{11}$ bilden sind

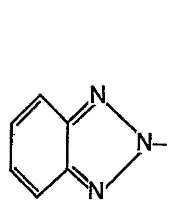

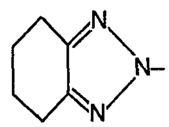

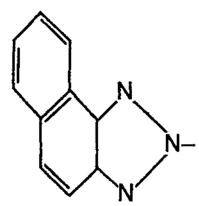

Geeignete Anionen sind ein Halogenid, Formiat, Acetat, Lactat, Alkylsulfat, Arylsulfat, Carbonat oder Bicarbonat.

Es ist als ausgesprochen überraschend zu bezeichnen, dass hier der Ringschluss durch einfaches Erhitzen erfolgt, da normalerweise bei derartigen Cyclisierungsreaktionen Katalysatoren, wie z.B. starke Basen, eingesetzt werden müssen (siehe z.B. DE-OS 2 238 628 = US-PS 3 864 333 sowie J. Chem. Soc. 1960, S. 1954).

Das erfindungsgemässe Verfahren wird zweckmässigerweise so durchgeführt, dass eine Verbindung der Formel (II) in einem organischen Lösungsmittel bei Temperaturen von 80–250°C, bevorzugt bei 110–200°C, erhitzt wird, bis die Wasserabspaltung beendet ist.

Als Lösungsmittel zur Durchführung dieser Reaktion eignen sich alle unter den Reaktionsbedingungen inerten Lösungsmittel, insbesondere aromatische Kohlenwasserstoffe wie Benzol, Toluol und Xylol; Halogenkohlenwasserstoffe wie Trichlorethan, Tetrachlorethylen, Chlorbenzol, Dichlorbenzol und Trichlorbenzol, ferner auch Nitrobenzol, Alkanole und offene oder cyclische Ether wie Butanol, Dibutylether, Diphenylether, Ethylenglykolmonomethylether, Ethylenglykolmonoethylether, Anisol oder Dioxan; Ketone wie Cyclohexanon oder Methylethylketon; Fettsäureamide wie Dimethylformamid oder Dimethylacetamid; Sulfoxide wie Dimethylsulfoxid und Carbonsäureester wie Essigester oder Essigsäurebutylester. Bei diesem Verfahren ist es nicht notwendig, dass sich die Verbindungen der Formel (II) in den angegebenen Solventien lösen; die Cyclisierung kann ebenso in Suspension vorgenommen werden.

Die Verbindungen der Formel (II) erhält man beispielsweise, indem man eine Verbindung der Formel

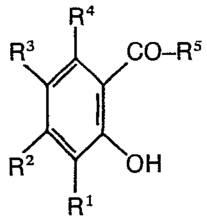

(III)

in der

$R^1$, $R^2$, $R^3$, $R^4$ und $R^5$ die oben angegebene Bedeutung haben, mit einer Verbindung der Formel

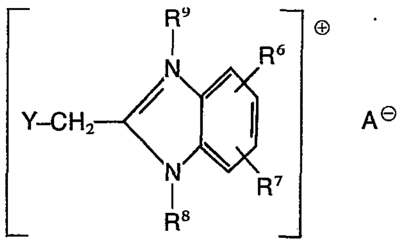

(IV)

in der

$R^6$, $R^7$, $R^8$, $R^9$, $A^{\ominus}$ die oben angegebene Bedeutung haben und

Y Chlor oder Brom bedeutet, in Gegenwart einer schwachen Base umsetzt.

Dabei wird zweckmässigerweise so vorgegangen, dass man die Verbindungen der Formeln (III) und (IV) in äquimolaren Mengen in einem organischen Lösungsmittel bei 20–120 °C, bevorzugt bei 40–90 °C, in Gegenwart von mindestens molaren Mengen einer Base umsetzt.

Als Lösungsmittel zur Durchführung dieser Reaktion eignen sich z. B. Alkohole wie Methanol, Ethanol oder Ethylenglykolmonomethylether, Ketone, wie z. B. Aceton, Methylethylketon ocer Methylisobutylketon, Fettsäureamide wie z. B. Dimethylformamid oder Dimethylacetamid, Sulfoxyde wie Dimethylsulfoxyd und Hexamethylphosphorsäuretriamid.

Als schwache Basen, die als säurebindende Mittel fungieren, können anorganische und organische Basen, bevorzugt Alkali-und Erdalkalicarbonate und -bicarbonate, beispielsweise Natriumcarbonat und Kaliumcarbonat, sowie tertiäre Amine, wie Triethylamin oder Pyridin, eingesetzt werden.

Falls die Verbindungen der Formel (III) als Alkali- bzw. Erdalkalisalze eingesetzt werden, kann auf die Zugabe von säurebindenden Mitteln verzichtet werden.

Die Verbindungen der Formel (III) sind bekannt bzw. nach literaturbekannten Verfahren leicht zugänglich (siehe z. B. H. Giesecke und J. Hocker, Liebigs Annalen der Chemie, 1978, 345).

Die Verbindungen der Formel (IV) werden nach dem in der US-PS 3 313 824 beschriebenen Verfahren durch Umsetzung von Halogenessigsäuren mit entsprechend substituierten o-Phenylendiaminen und anschliessende Quaternierung mit einer Verbindung der Formel

$$R^9\text{–}A \qquad (V)$$

in der

$R^9$ und A die oben angegebene Bedeutung haben, gewonnen.

Eine bevorzugte Verfahrensvariante zur Herstellung von Verbindungen der Formel (I) besteht darin, dass die entsprechende unquaternierte Verbindung der Formel (II) zunächst mit einer Verbindung der Formel (V) in einem geeigneten Lösungsmittel quaterniert und anschliessend ohne Zwischenisolierung bei Temperaturen von 80–250 °C, bevorzugt bei 110–200 °C, cyclisiert wird.

Als Quaternierungsmittel der Formel (V) verwendet man vorzugsweise Dialkylsulfate wie Dimethyl- und Diethylsulfat, Alkyl-halogenide wie Methylchlorid, Ethyl-, Propyl- und Butyliodid oder -bromid, Allylchlorid oder -bromid, Crotylchlorid oder -bromid, sowie Alkylbenzolsulfonate wie p-Methyl-, Ethyl-oder Chlorbenzolsulfonat. Wenn es gewünscht ist, Verbindungen der Formel (I) herzustellen, die mit einem Benzylrest quaterniert sind, so verwendet man für die Benzylierung vorzugsweise Benzylhalogenide wie Benzylchlorid. Weitere Quaternierungsmittel sind z. B. $BrCH_2CH_2OH$, $BrCH_2CHOHCH_3$, Halogenessigsäurederivate wie $ClCH_2CO_2CH_2CH_3$, $BrCH_2COOH$, $BrCH_2COOCH_3$, $ClCH_2CN$, $ClCH_2CONH_2$, $ClCH_2CONHCH_3$ und $ClCH_2CON(CH_3)_2$ sowie Ethylenoxid oder Propylenoxid in Gegenwart geeigneter Anionen wie z. B. der Ameisen-, Essig- oder Milchsäure.

Als Lösungsmittel für dieses Eintopfverfahren eignen sich im allgemeinen alle inerten Lösungsmittel. Bevorzugt sind solche, die das Ausgangsprodukt lösen und aus denen sich das Endprodukt sofort ausscheidet. Beispielsweise seien genannt: aromatische Kohlenwasserstoffe wie Benzol, Toluol und Xylol; Halogenkohlenwasserstoffe wie Trichlorethan, Tetrachlorethylen, Chlorbenzol oder Dichlorbenzol, ferner auch Nitrobenzol, Alkanole und offene oder cyclische Ether wie Butanol, Dibutylether, Ethylenglykolmonomethylether, Ethylenglykolmonoethylether, Anisol oder Dioxan; Ketone wie Cyclohexanon oder Methylethylketon; Fettsäureamide wie Dimethylformamid oder Dimethylacetamid; Sulfoxide wie Dimethylsulfoxid und Carbonsäureester wie Essigester oder Essigsäure-butylester. Zuweilen ist es vorteilhaft, überschüssiges Alkylierungsmittel als Lösungsmittel zu verwenden.

Die nach den erfindungsgemässen Verfahren hergestellten Produkte sind z. T. bekannte wertvolle optische Aufheller (vgl. DE-OS 2 031 774 und DE-OS 2 159 469).

Bevorzugte Verfahrensprodukte entsprechen der Formel (I), worin

$R^1$, $R^3$, $R^4$ Wasserstoff, Methyl, Ethyl, Methoxy, Chlor,

$R^2$ Wasserstoff, Chlor, $C_1$–$C_4$-Alkyl, $C_1$–$C_4$-Alkoxy, durch $C_1$–$C_4$-Alkyl und/oder $C_1$–$C_4$-Alkoxy substituiertes Phenyl oder ein Substituent der allgemeinen Formel

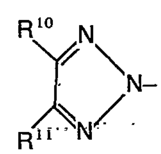

in der

$R^{10}$ $C_1$–$C_4$-Alkyl, $C_1$–$C_4$-Alkoxy, ein Phenylrest,

der gegebenenfalls durch $C_1$–$C_4$-Alkyl, Phenyl, $C_1$–$C_4$-Alkoxy oder Chlor substituiert sein kann, Benzyl oder Styryl,
$R^{11}$ Wasserstoff, Cyan, Carboxy, $C_1$–$C_4$-Alkylcarbonylamino, Benzoylamino oder $R^{10}$,
$R^{10}$ und $R^{11}$ zusammen einen ankondensierten Naphthalinring oder Benzolring, der gegebenenfalls durch $C_1$–$C_4$-Alkyl und/oder $C_1$–$C_4$-Alkoxy substituiert sein kann,
$R^5$ Wasserstoff, Alkyl mit 1–4 C-Atomen, vorzugsweise Methyl oder gegebenenfalls mit Methyl und/oder Methoxy substituiertes Phenyl,
$R^6$ Wasserstoff, Alkyl mit 1–4 C-Atomen, vorzugsweise Methyl, Methoxy, Chlor, Alkylsulfonyl mit 1–4 C-Atomen, Cyan, COOH, $SO_3H$, $COOR^8$, $SO_3R^8$ oder $CON(R^5)_2$,
$R^7$ Wasserstoff, Methyl, Methoxy cder Chlor,
$R^8$ Alkyl mit 1–4 C-Atomen, vorzugsweise Methyl, Hydroxyalkyl mit 2–4 Kohlenwasserstoffatomen, Cyanethyl, gegebenenfalls durch Chlor, Methyl oder Methoxy substituiertes Phenyl, Cyclohexyl oder Benzyl,
$R^9$ Alkyl mit 1–4 C-Atomen, gegebenenfalls mit Hydroxy oder Alkoxy mit 1–4 C-Atomen substituiert, vorzugsweise Methyl, Benzyl, gegebenenfalls mit Chlor oder Methoxy substituiert, einen Rest $-CH_2-CN$, $-CH_2-CONH_2$, $-CH_2-COOR^8$ oder Cyclohexyl,
$A^-$ ein Halogenid, Formiat, Acetat, Lactat, $CH_3SO_4^-$, $C_2H_5SO_4^-$, $C_6H_5SO_3^-$, $p-CH_3-C_6H_4-SO_3^-$, $p-Cl-C_6H_4-SO_3^-$, Carbonat oder Bicarbonat.

Beispiel 1

10 g der Verbindung der Formel

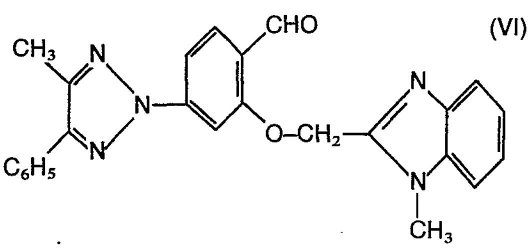

(VI)

werden in 100 ml 1,2-Dichlorbenzol 7 Std. bei 170 °C gerührt, wobei das entstehende Wasser abdestilliert wird. Anschliessend wird mit einem Eisbad gekühlt, abgesaugt und der Filterkuchen mit 20 ml Methanol gewaschen. Nach dem Trocknen erhält man 8,5 g (89% d.Th.) der Verbindung der Formel

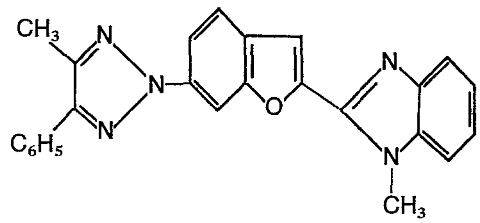

(VII)

vom Fp. 222 – 4 °C.

Die Verbindung der Formel (VI) gewinnt man auf folgendem Wege:

14,0 g 4-(4-Methyl-5-phenyl-1,2,3-triazol-2-yl)-salicylaldehyd, 9,9 g 2-Chlormethyl-1-methylbenzimidazol und 6,4 g wasserfreies Natriumcarbonat werden in 100 ml Dimethylformamid 5 Std. bei 70 °C gerührt. Dann versetzt man mit 100 ml Wasser, saugt bei Raumtemperatur ab und wäscht mit 100 ml Wasser nach. Man trocknet bei 100 °C und erhält so 20,7 g (98% d.Th.) der Verbindung der Formel (VI) vom Fp. 216 – 18 °C. Aus Methylglykol umkristallisiert schmilzt das Produkt bei 236 °C.

10 g der Verbindung der Formel (VI) werden in 90 ml 1,2-Dichlorbenzol bei 140 °C gelöst und innerhalb von 10 Min. eine Lösung von 3,4 g Dimethylsulfat (97%) in 10 ml 1,2-Dichlorbenzol zugetropft. Anschliessend wird 4 Std. bei 140 °C gerührt und das entstehende Wasser laufend abdestilliert. Man lässt auf 80 °C abkühlen, saugt ab und wäscht mit 20 ml Aceton. Nach dem Trocknen bei 100 °C erhält man 11,6 g (93%d.Th.) der Verbindung der Formel

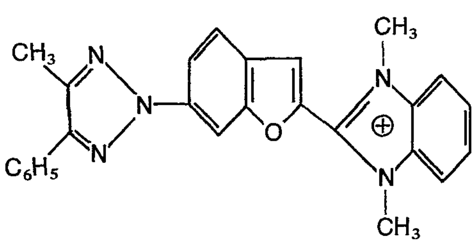

$CH_3OSO_3^{\ominus}$ (VIII)

vom Fp. 260 – 1 °C.

Verwendet man anstelle von Dimethylsulfat Diethylsulfat und verfährt im übrigen wie vorstehend beschrieben, so erhält man die Verbindung der Formel

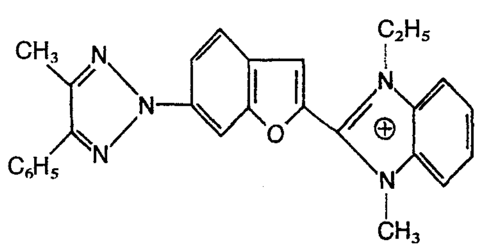

$C_2H_5OSO_3^{\ominus}$ (IX)

Fp. 195 – 7 °C
Ausbeute: 91% d.Th.

Beispiel 2

Unter Einsatz der Phenolether der Formeln

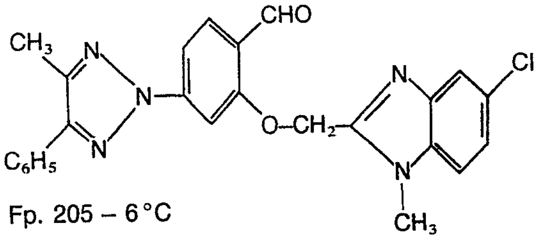

(X)

Fp. 205 – 6 °C

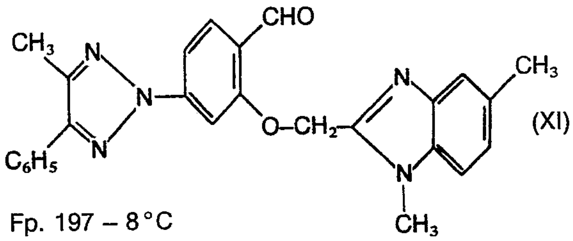

(XI)

Fp. 197 – 8 °C

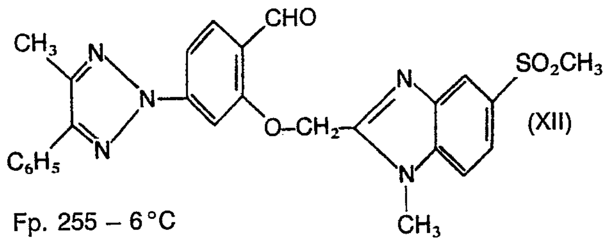

(XII)

Fp. 255 – 6 °C

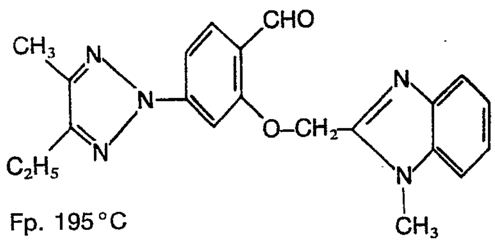

(XIII)

Fp. 195 °C

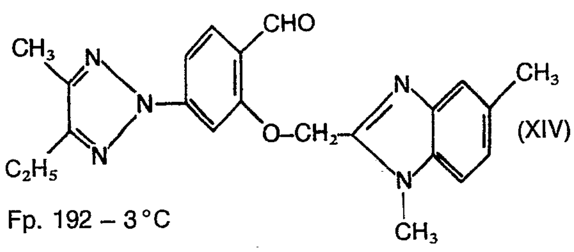

(XIV)

Fp. 192 – 3 °C

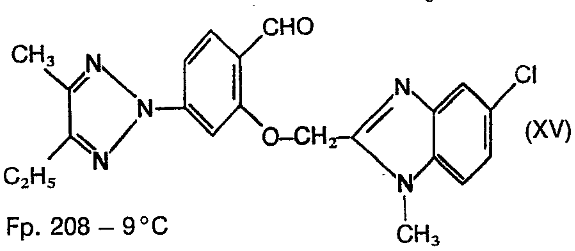

(XV)

Fp. 208 – 9 °C

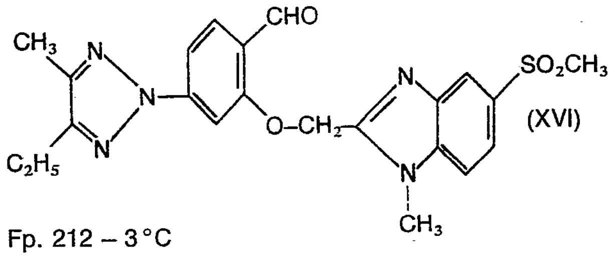

(XVI)

Fp. 212 – 3 °C

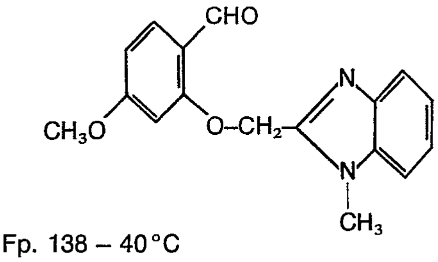

(XVII)

Fp. 138 – 40 °C

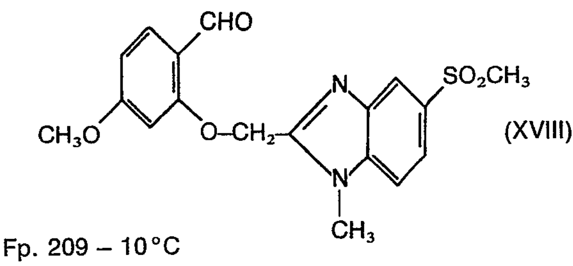

(XVIII)

Fp. 209 – 10 °C

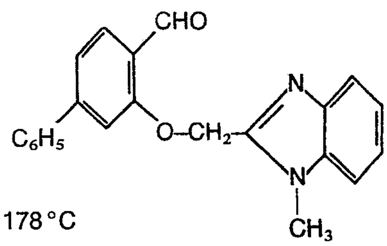

(XIX)

Fp. 178 °C

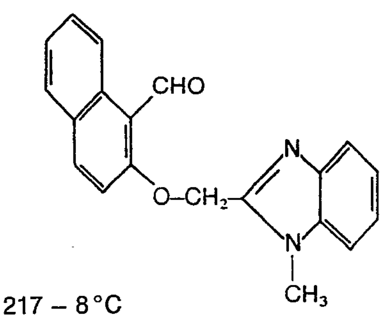

(XX)

Fp. 217 – 8 °C

werden in Analogie zum Beispiel 1 folgende Verbindungen erhalten:

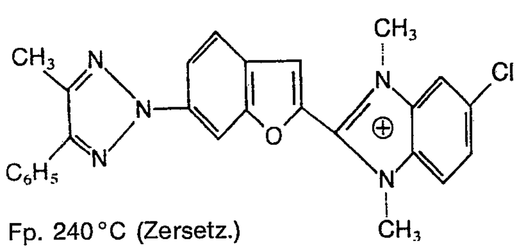

$CH_3O{-}SO_3^{\ominus}$ (XXI)

Fp. 240 °C (Zersetz.)

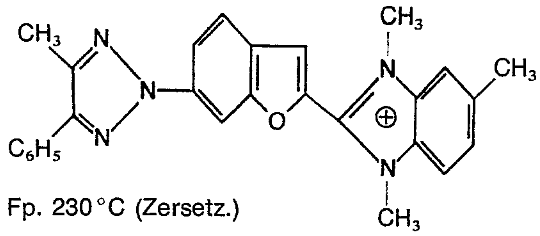

Fp. 230 °C (Zersetz.)

$CH_3O-SO_3^{\ominus}$ (XXII)

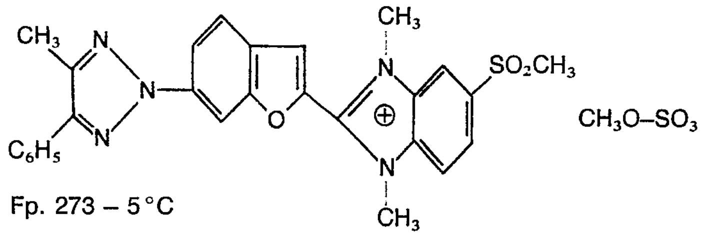

Fp. 273 – 5 °C

$CH_3O-SO_3^{\ominus}$ (XXIII)

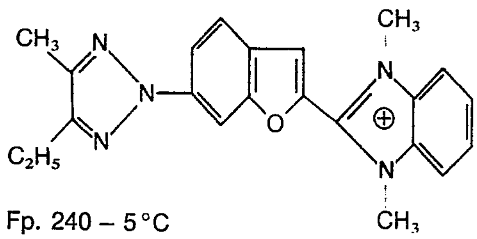

Fp. 240 – 5 °C

$CH_3O-SO_3^{\ominus}$ (XXIV)

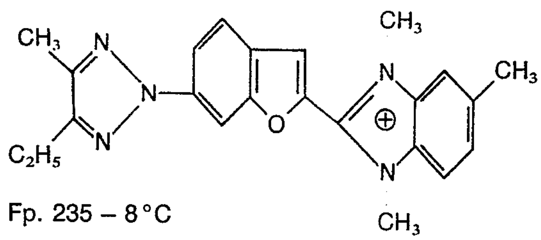

Fp. 235 – 8 °C

$CH_3O-SO_3^{\ominus}$ (XXV)

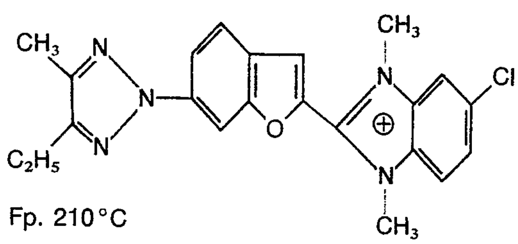

Fp. 210 °C

$CH_3O-SO_3^{\ominus}$ (XXVI)

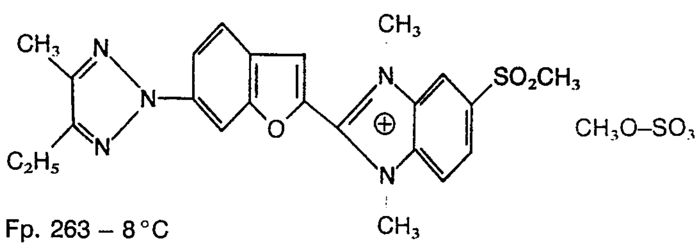

Fp. 263 – 8 °C

$CH_3O-SO_3^{\ominus}$ (XXVII)

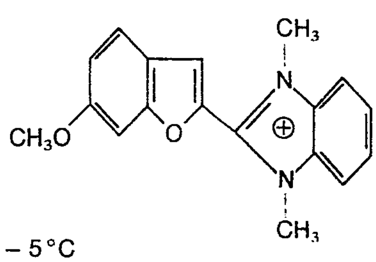

Fp. 224 – 5 °C

$CH_3O-SO_3^{\ominus}$ (XXVIII)

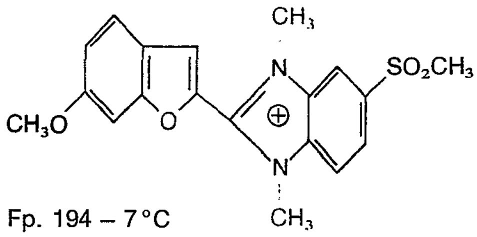

Fp. 194 – 7 °C

$CH_3O–SO_3^{\ominus}$ (XXIX)

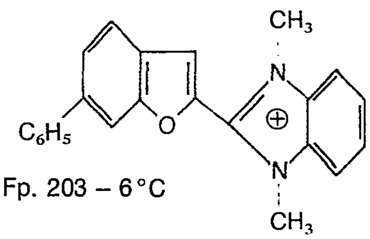

Fp. 203 – 6 °C

$CH_3O–SO_3^{\ominus}$ (XXX)

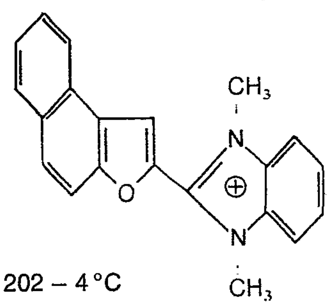

Fp. 202 – 4 °C

$CH_3O–SO_3^{\ominus}$ (XXXI)

Beispiel 3

Wenn man die Verbindung der Formel (VI) durch die Verbindungen der Formeln

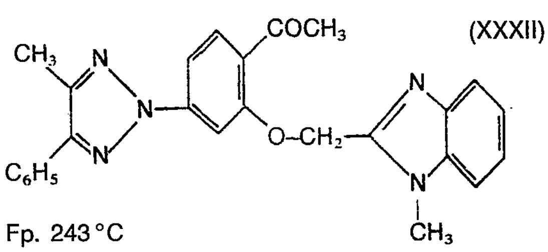

(XXXII)

Fp. 243 °C

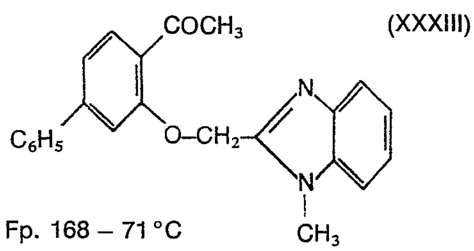

(XXXIII)

Fp. 168 – 71 °C

ersetzt und im übrigen so verfährt, wie in Beispiel 1 beschrieben, erhält man die Benzimidazolyl-benzofurane der Formeln

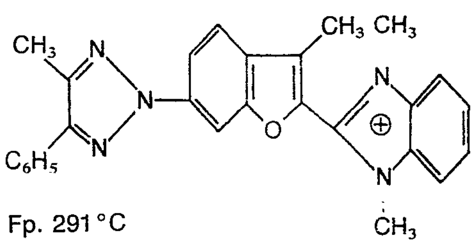

Fp. 291 °C

$CH_3O–SO_3^{\ominus}$ (XXXIV)

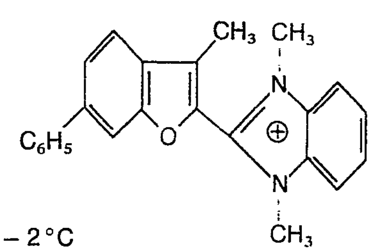

Fp. 151 – 2 °C

$CH_3O–SO_3^{\ominus}$ (XXXV)

**Patentansprüche**

1. Verfahren zur Herstellung von Verbindungen der Formel

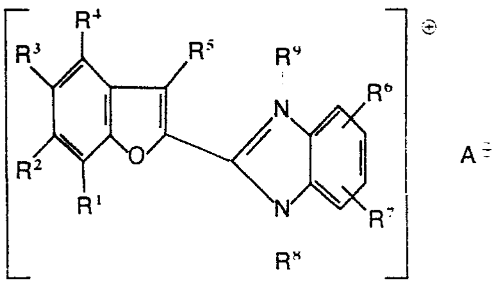

(I)

worin

$R^1$, $R^3$, $R^4$ Wasserstoff, Alkyl, Alkoxy oder Halogen

$R^2$ Wasserstoff, Halogen, Alkyl, Alkoxy, Aryl oder einen Substituenten der allgemeinen Formel

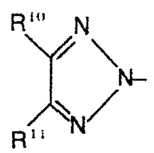

in der

$R^{10}$ Aryl, Alkyl, Aralkyl, Styryl oder Alkoxy,

$R^{11}$ Wasserstoff, CN, COOR, CONRR' oder $R^{10}$ und zwei benachbarte

$R^1$, $R^2$, $R^3$, $R^4$, $R^{10}$, $R^{11}$ zusammen einen anellierten hydroaromatischen oder aromatischen Ring,

$R^5$ Wasserstoff, Aryl, Alkyl,

$R^6$ Wasserstoff, Halogen, Alkyl, Alkoxy, Alkylsulfonyl, Arylsulfonyl, CN, $CF_3$, COOR, $SO_3R$, CONRR' oder $SO_2NRR'$,

$R^7$ Wasserstoff, Halogen, Alkyl, Alkoxy,

$R^8$ Wasserstoff, Alkyl, Cycloalkyl, Alkenyl, Aryl oder Aralkyl,

$R^9$ Cycloalkyl, Alkenyl, Alkyl, Aralkyl,

$A^\ominus$ ein farbloses Anion einer anorganischen oder organischen Säure.

R Alkyl und

R' R oder H, wobei die vorstehend genannten Kohlenwasserstoff- und Alkoxyreste sowie die gegebenenfalls anellierten Ringsysteme durch in der Aufhellerchemie übliche nichtionische Substituenten substituiert sein können,

dadurch gekennzeichnet, dass man eine Verbindung der Formel

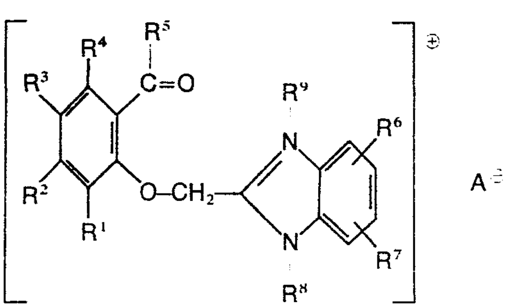

(II)

worin $R^1$, $R^2$, $R^3$, $R^4$, $R^5$, $R^6$, $R^7$, $R^8$, $R^9$, A die oben genannte Bedeutung haben, in einem inerten Lösungsmittel ohne Katalysatorzusatz erhitzt.

2. Verfahren nach Anspruch 1, dadurch gekennzeichnet, dass man die Reaktion bei 110–200 °C durchführt, bis die Wasserabspaltung beendet ist.

3. Verfahren nach Anspruch 1, dadurch gekennzeichnet, dass man Quaternierung und Cyclisierung ohne Zwischenisolierung vornimmt.

**Claims**

1. Process for the preparation of compounds of the formula

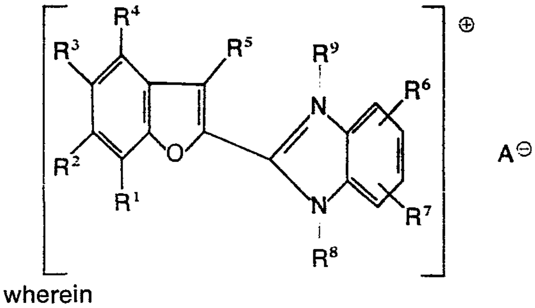

(I)

wherein

$R^1$, $R^3$ and $R^4$ hydrogen, alkyl, alkoxy or halogen,

$R^2$ hydrogen, halogen, alkyl, alkoxy, aryl or a substituent of the general formula

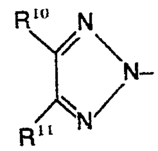

in which

$R^{10}$ aryl, alkyl, aralkyl, styryl or alkoxy and

$R^{11}$ hydrogen, CN, COOR, CONRR' or $R^{10}$ and two adjacent radicals from the group

$R^1$, $R^2$, $R^3$, $R^4$, $R^{10}$ and $R^{11}$ together a fused-on hydroaromatic or aromatic ring,

$R^5$ hydrogen, aryl or alkyl,

$R^6$ hydrogen, halogen, alkyl, alkoxy, alkyl-sulphonyl, arylsulphonyl, CN, $CF_3$, COOR, $SO_3R$, CONRR' or $SO_2NRR'$,

$R^7$ hydrogen, halogen, alkyl or alkoxy,

$R^8$ hydrogen, alkyl, cycloalkyl, alkenyl, aryl or aralkyl,

$R^9$ cycloalkyl, alkenyl, alkyl or aralkyl,

$A^\ominus$ a colourless anion of an inorganic or organic acid,

R alkyl and

R' R or H,

and wherein the abovementioned hydrocarbon radicals and alkoxy radicals and the optionally fused-on ring systems can be substituted by non-ionic substituents customary in brightener chemistry, characterised in that a compound of the formula

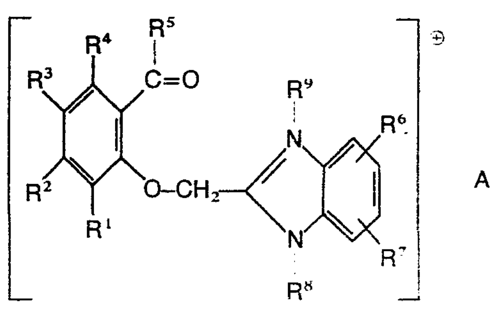

(II)

wherein
$R^1$, $R^2$, $R^3$, $R^4$, $R^5$, $R^6$, $R^7$, $R^8$, $R^9$ and A have the abovementioned meaning,
is heated in an inert solvent, without the addition of a catalyst.

2. Process according to claim 1, characterised in that the reaction is carried out at 110–200 °C until the splitting off of water has ended.

3. Process according to claim 1, characterised in that quaternisation and cyclisation are carried out without intermediate isolation.

**Revendications**

1. Procédé de production de composés de formule:

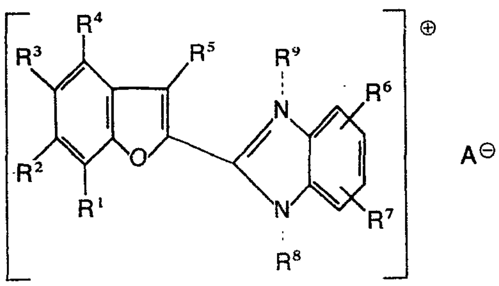

(I)

dans laquelle:
$R^1$, $R^3$, $R^4$ représentent de l'hydrogène, un groupe alkyle, un groupe alkoxy ou un halogène
$R^2$ représente de l'hydrogène, un halogène, un groupe alkyle, alkoxy, aryle ou un substituant de formule générale:

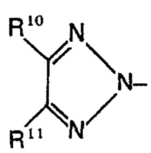

dans laquelle:
$R^{10}$ est un groupe aryle, alkyle, aralkyle, styryle ou alkoxy,
$R^{11}$ est un atome d'hydrogène, un groupe CN, COOR, CONRR' ou $R^{10}$ et
deux groupes $R^1$, $R^2$, $R^3$, $R^4$, $R^{10}$, $R^{11}$ voisins forment ensemble un noyau hydro-aromatique ou aromatique condensés,
$R^5$ est de l'hydrogène ou un groupe aryle ou alkyle,
$R^6$ est de l'hydrogène, un halogène ou un groupe alkyle, alkoxy, alkylsulfonyle, arylsulfonyle, CN, $CF_3$, COOR, $SO_3R$, CONRR' ou $SO_2NRR'$,
$R^7$ est de l'hydrogène, un halogène ou un groupe alkyle ou alkoxy,
$R^8$ est de l'hydrogène ou un groupe alkyle, cycloalkyle, alcényle, aryle ou aralkyle,
$R^9$ est un groupe cycloalkyle, alcényle, alkyle, aralkyle,
$A^{\ominus}$ est un anion incolore d'un acide inorganique ou organique,
R est un groupe alkyle et
R' représente R ou l'hydrogène, les restes hydrocarbonés et alkoxy mentionnés ci-dessus ainsi que les noyaux éventuellement condensés pouvant être substitués par des substituants non ioniques classiques dans la chimie des azurants, caractérisé en ce qu'on chauffe un composé de formule:

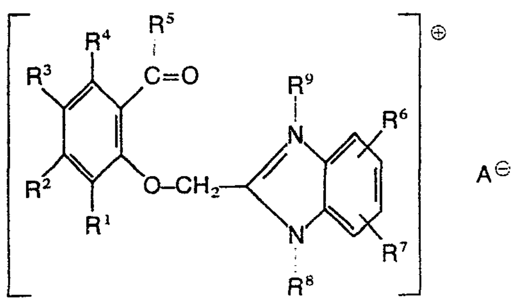

(II)

dans laquelle $R^1$, $R^2$, $R^3$, $R^4$, $R^5$, $R^6$, $R^7$, $R^8$, $R^9$, A ont la définition donnée ci-dessus, dans un solvant inerte sans addition de catalyseur.

2. Procédé suivant la revendication 1, caractérisé en ce qu'on conduit la réaction à 110–200 °C jusqu'à ce que l'élimination d'eau soit terminée.

3. Procédé suivant la revendication 1, caractérisé en ce qu'on effectue une quaternisation et une cyclisation sans isolement intermédiaire.